# EUROPÄISCHE PATENTSCHRIFT

(11) **EP 1 734 895 B1**
(45) Veröffentlichungstag und Bekanntmachung des Hinweises auf die Patenterteilung: **02.07.2008**
(21) Anmeldenummer: 05740680.3
(22) Anmeldetag: 12.04.2005
(51) Int. Cl.: A61F 2/00

(54) **IMPLANTIERBARES SCHLIESSMUSKELPROTHESENSYSTEM, INSBESONDERE ZUM EINSATZ IM BEREICH DES ANALKANALS**
IMPLANTABLE MUSCLE CLOSING PROSTHESIS SYSTEM, IN PARTICULAR IN THE ANAL CHANNEL AREA
SYSTEME IMPLANTABLE DE PROTHESE DE MUSCLE DE FERMETURE, EN PARTICULIER DANS LA ZONE DU CANAL ANAL

(30) Priorität: 15.04.2004 DE 102004018807
(43) Veröffentlichungstag der Anmeldung: 27.12.2006
(73) Patentinhaber: Universitätsklinikum Freiburg, 79095 Freiburg (DE)
(72) Erfinder: SCHRAG, Hans-Jürgen, 79877 Friedenweiler (DE)
(74) Vertreter: Rösler, Uwe
(86) Internationale Anmeldenummer: PCT/DE2005/000640
(87) Internationale Veröffentlichungsnummer: WO 2005/099618

(56) Entgegenhaltungen:
- EP-A- 0 028 962
- WO-A-01/45488
- WO-A-01/47431
- WO-A-01/58390
- WO-A-92/16162
- DE-A1- 3 539 498
- DE-A1- 10 013 519
- DE-A1- 19 845 292
- US-A- 4 428 365
- US-A1- 2001 041 823

## Beschreibung

Die Erfindung betrifft ein verbessertes, implantierbares Sphinkterprothesensystem zur Therapie der hochgradigen Stuhlinkontinenz, das durch seine hochintegrative Konzeption Platz sparend um den Analkanal implantiert werden kann, wobei hierzu das Dokument DE 198 45 292 A den nächstliegenden Stand der Technik bildet.

Der derzeit klinisch relevante und im Schrifttum beschriebene Acticon^{®} Neosphinkter zur Behandlung der hochgradigen Stuhlinkontinenz besteht aus drei separaten, in unterschiedliche anatomische Bereiche zu implantierenden Funktionsbausteinen: einem aufpumpbaren, manschettenartig um den Darm zu justierenden Ring, der den Analkanal komprimiert, einem separaten Druckspeicher für das Kompressionsmedium und einer unidirektionalen Pumpe, die beide Bausteine verbindet und das Kompressionsmedium aus der Kompressionsmanschette zurück in den Druckspeicher pumpt (Wong WD, et al, Disease of Colon and Rectum 2002, 9:1139-1153). Die Anordnung der Funktionsbausteine setzt eine komplexe Chirurgie voraus und resultiert in Komplikations- und Explantationsraten um 40%. Eine kompaktere Schließmuskelvorrichtung, bei der für den Kompressionsvorgang eine Änderung der Viskosität und Dichte einer kolloidalen magnetorheologischen Flüssigkeit ausgenutzt wird (US 5509888 A), ist bislang ohne klinische Relevanz und entspricht nicht den Merkmalen des kennzeichnenden Teils in Anspruch 1. Verschiedene, aus mehreren, sich peristaltisch bewegenden Abdichtelementen, bestehende Vorrichtungen zum Verschluss eines Darmendes (DE 100 23 634 C2; DE 197 32 982 A1) sind nicht im Bereich des Analkanals einsetzbar und unterscheiden sich ebenso wie die aus der Urologie bekannten Systeme zum Verschluß der Harnröhre (DE 90 10 783 U1; EP 02 02 815 A2; EP 03 48 114 A1) wesentlich von der in Anspruch 1 bezeichneten Erfindung.

Des weiteren handelt es sich bei den im Schrifttum aufgeführten Kompressionsmanschetten um hochelastische Blähkörper, die im Rahmen der In- bzw. Deflation im wesentlichen einer Dehnung unterliegen (Luftballonprinzip). Die Erzielung einer zirkulären, symmetrischen und effizienten Lumenkompression mit kleinsten Volumen gegen hohe Drücke, wie sie im Rahmen einer effizienten Kontinenzkompression im Bereich des Analkanals auftreten können, sind aufgrund der starken Verformung der Blähkörper enge Grenzen gesetzt. Zudem beeinflusst während der Inflation der bereits zu Beginn bestehende, hohe Druckgradient im Blähkörper das Leistungsspektrum einer zum Einsatz kommenden Mikropumpe äußerst negativ.

Die Neuerung bezieht sich somit auf eine Schließmuskelprothese zur willkürlichen Erzielung der Stuhlkontinenz bzw. Defäkation, die aufgrund ihrer einzigartig kompakten Bauart und Funktion wesentlich vereinfacht perianal, über den M. sphincter ani externus im Bereich des Analkanals oder konventinell, mittels Laparotomie oder laparoskopisch in Höhe des kleinen Beckenausgangs implantiert werden kann und sämtliche für die Funktion notwendigen Bausteine integriert.

Erfindungsgemäß gelöst wird dieses Problem durch den Aufbau von einer Kompressionseinheit, gemäß den kennzeichnenden Merkmalen des Anspruchs 1.

Die nachgeordneten Patentansprüche zeigen Weiterbildungen bzw. vorteilhafte Ausführungsbeispiele.

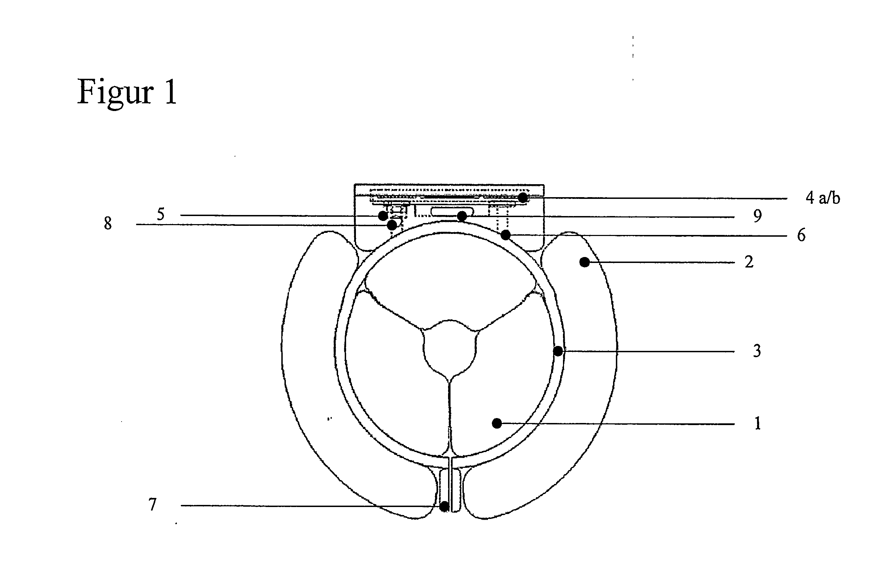

Die Funktion der Schließmuskelprothese wird durch die Ausführungsbeschreibung anhand von Figur 1 erläutert.

### Bezugszeichen

- 1: Kompressionscuff
- 2: Reservoircuff

- 3: Trägerring
- 4: Mikropumpe
- 4a/b: Sperrventile
- 5: Drucksensor
- 6: Transmissionsleitung
- 7: Schnellverschluss
- 8: Port
- 9: Anschluss für externe Energiequelle/Steuerungselektronik

Im Detail zeigt Figur 1 die vollständige Kompressionseinheit im Coronarschnitt, mit Kompressions (1)- und Reservoircuffs (2), dem Trägerring (3) und der integrierten Mikropume (4). Die geformten Hohlkörper der Kompressions- und Reservoircuffs sind in einem nicht evakuierten, bzw. inflatierten Zustand dargestellt. Der Kompressionscuff (1) ist in konkaver, dreikammeriger Ausführung abgebildet.

Der Einsatz einer bidirektionalen Mikropumpe (4) mit integrierten Sperrventilen (4a/b), in Figur 1 als Piezopumpe ausgeführt, ermöglicht die Reduktion der bislang üblichen, großen und separat zu implantierenden Druckausgleichs- bzw. Reservoirbehälter. Das Volumen muß auf diese Weise lediglich dem Kompressionsvolumen entsprechen, so dass der Reservoircuff (2) an der Aussenseite des Trägerringes (3) angebracht werden kann.

Eine weitere Neuerung besteht nach Anspruch 1 in der Ausführung der Kompressions- (1) und Reservoircuffs (2), die als evakuierbare Hohlkörper z.B. aus hierfür hervorragend geeignetem Polyurethan gefertigt sind. Die In-bzw. Deflation basiert somit hauptsächlich auf einer Materialstauchung und Verformung eines Hohlkörpers mit definiertem Volumen bei weitgehend konstanter Wandstärke und nicht auf einer, wie üblicherweise bei den herkömmlichen z.B. aus Silikon gefertigten Kompressionskörpern bestehenden, Materialdehnung.

Der Vorteil besteht neben der geringeren Materialbeanspruchung in der Eliminierung des initialen Dehnungswiderstandes des Kompresionscuffs (1), woraus sich ein Hysteresephänomen des intracuffalen Druckes während der Kompressionfüllung ableitet. Diese Konstellation, in Kombination mit den aus der integrierten Systemkonzeption resultierenden, minimalen Totraumvolumen der Transmissionsleitungen (6), wirkt sich positiv auf die Leistungscharakteristik und das Energiemanagement der zu verwendenden Mikropumpe (4) aus.

Der Trägerring (3), und die beidseitig angebrachten Cuffs (1,2) (idealerweise aus Polyurethan gefertigt) erlauben die Öffnung des Ringes zur Implantation um 180°. Der Ring kann durch einen Schnellverschluß (7) mechanisch oder elektromagnetisch erfolgen..

Nach Implantation der Prothese mit primär evakuierten Cuffs, erfolgt die Füllung der Reservoircuffs (2) über den Port (8). Die Aktivierung der Mikropumpe (4) bewirkt die Füllung der Kompressionscuffs (1) aus den Reservoircuffs (2) über die integrierten Transmissionsleitungen (6). Der Verschluss des Analkanals ist hergestellt (Kontinenzleistung).
Eine Umkehr der Fluid- bzw. Lufttransmission bewirkt die Entleerung der Kompressionscuffs (1) (Defäkation). Der integrierte Drucksensor (5) ermöglicht eine gewebeprotektive Druckregulation und ist zusätzlich als Indikator für eine bevorstehende Defäkation nutzbar (Zunahme des intracuffalen Drucks bei anstehender Stuhlsäule). Ein separates Patientenprogrammiergerät dient zur willkürlichen Aktivierung der Kontinenzleistung bzw. der Defäkation (Funktion Ein/Aus, bzw. druck-/volumenadaptierte Regulation in Abhängigkeit der Stuhlkonsistenz und innerhalb eines vom Arzt vorgegebenen Druckbereiches). In diesem Fall wird mit dem Patientenprogrammiergerät ein subcutan implantierter Empfänger z.B. telemetrisch oder per Funk angesteuert, wobei der Empfänger über eine Kabelverbindung durch geeignete Anschlüsse (9) mit der Sphinkterprothese verbunden ist. Die Ansteuerung kann auch mit einem in die Prothese integrierten funkferngesteuerten Modul erfolgen, wodurch die Funktionsbausteine um den subcutanen Empfänger und die Kabelverbindung reduziert würden.

## Patentansprüche

1. Implantierbares Schließmuskelprothesensystem zum Öffnen und Schließen des Analkanals bzw. eines Hohlorgansegments,
**dadurch gekennzeichnet,**
**dass** ein primär evakuierter, elastischer und als Hohlkörper ausgeformter Kompressionscuff (1) an der Innenseite eines elastischen Trägerrings (3) über eine integrierte oder nicht integrierte Mikropumpe (4) beliebiger Bauart, mit einem ebenfalls primär evakuierten, an der Außenseite des Trägerrings angebrachten, elastischen und ebenfalls als Hohlkörper ausgeformtem Reservoircuff (2) verbunden ist, dass der jeweils als Hohlkörper ausgeformte Kompressionscuff (1) und der Reservoircuff (2) im Wege der In- und Deflation einer Materialstauchung oder Verformung bei weitgehend konstanter Wandstärke unterliegen, wobei über einen Port das Transmissionsfluid oder ein Gas oder Gasgemisch in den evakuierten Reservoircuff (2) injezierbar ist und über die Mikropumpe (4) zwischen evakuiertem Reservoircuff (2) und Kompressionscuff (1) transferiert wird und die Mikropumpe (4) von einem externen Programmiergerät magnetisch, telemetrisch oder per Induktionsspule über einen zwischengeschalteten und separat zu implantierenden Empfänger, der mit der Prothese über ein Kabel verbunden ist oder per Funk direkt ansteuerbar ist.

2. Schließmuskelprothesensystem nach Anspruch 1,
**dadurch gekennzeichnet,**
**dass** der als geformter Hohlkörper vorliegende Kompressionscuff (1) an der Innenseite des Trägerrings mit der zum komprimierenden Hohlorgan gewandten Fläche konvex oder konkav ausgebildet sein kann und aus zwei oder mehr miteinander in Verbindung stehenden Einzelkammern besteht, die sich gleichzeitig oder in versetzter zeitlicher Reihenfolge, symmetrisch oder asymmetrisch gegeneinander füllen lassen.

3. Schließmuskelprothesensystem nach Anspruch 1,
**dadurch gekennzeichnet, dass** zur Reduktion des Kompressionsvolumens innerhalb der als Hohlkörper ausgeformten Kompressionscuffs (1) elastische, komprimierbare Füllkissen, z.B. Schaumstoff, eingebracht sein können.

4. Schließmuskelprothesesystem nach Anspruch 1 bis 3,
**dadurch gekennzeichnet, dass** der an der Außenseite des Trägerrings angebrachte Reservoircuff (2) aus einer oder mehreren miteinander in Verbindung stehenden, geformten Hohlkammern besteht.

5. Schließmuskelprothesensystem nach Anspruch 1 bis 4,
**dadurch gekennzeichnet, dass** der als Hohlkörper geformte Reservoircuff (2) allseits von einer flexiblen und mit dem Trägerring verbundenen Hülle geschützt sein kann, die jedoch nicht in unmittelbarer Verbindung zu diesem Hohlkörper steht und somit im Rahmen der Deflation des Hohlkörpers nicht evakuiert werden kann und somit als Gewebeexpander dient.

6. Schließmuskelprothesensystem nach Anspruch 1 bis 5,
**dadurch gekennzeichnet, dass** zwischen dem Kompressionscuff (1) und dem Reservoircuff (2) mindestens ein oder mehrere Ventile zur Absperrung der Fluidtransmission angeordnet sind oder aufgrund der Bauart der Mikropumpe (4) in diese integriert sind.

7. Schließmuskelprothesensystem nach Anspruch 1,
**dadurch gekennzeichnet, dass** die Mikropumpe (4) nicht unmittelbar in die Prothese integriert sein muß, sondern in beliebiger Bauart bidirektional oder unidirektional gesteuert oder als manuelle unidirektionale Pumpe in einen anderen anatomischen Raum ausgelagert sein kann und in diesem Fall über eine Transmissionsleitung (6) mit dem Kompressions (1)- und Reservoircuff(2) verbunden ist.

8. Schließmuskelprothesensystem nach Anspruch 1 und 7,
**dadurch gekennzeichnet, dass** bei unidirektionaler Bauart der Pumpe, die Kompressionsflüssigkeit aktiv vom Reservoircuff (2) in den Kompressionscuff(1) gepumpt wird und die Entleerung des Kompressionscuff (1) passiv aufgrund des bestehenden Druckgradienten zwischen diesem und dem evakuierten Reservoircuff (2) sowie aufgrund des Druckes, der während der Defäkation durch die Stuhlsäule auf den Kompressionscuff (1) ausgeübt wird, stattfindet.

## Claims

1. An implantable muscle closing prosthesis system for opening and closing the anal canal or a hollow organ segment,
**characterized by** an elastic compression cuff (1) which may be primary evacuated, and is molded as a hollow body on the interior of an elastic support ring (3), connected via an integrated or nonintegrated micropump (4) of arbitrary construction to an elastic reservoir cuff (2), which may also be primary evacuated and is also molded as a hollow body attached to the exterior of the support ring; that the respective compression cuff (1) as deformed hollow body, and the reservoir cuff (2) lie in the way of in- and deflation of material dipping or deforming with extensive and constant wall strength, whereby the transfer fluid or a gas or gas mixture is injectable into the reservoir cuff (2), which is evacuated, via a port and transferred via the micropump (4) between evacuated reservoir cuff (2) and compression cuff (1) and the micropump (4) able to be activated magnetically, telemetrically, or by induction coil by an external programming device via an interposed receiver to be implanted separately, which is connected to the prosthesis via a cable, for example, or directly by radio.

2. The muscle closing prosthesis system according to Claim 1,
**characterized by** the compression cuff (1) provided as a molded hollow body on the interior of the support ring, which may be implemented having the surface facing toward the hollow organ to be compressed as convex or concave, and comprising multiple individual chambers connected to one another, which may be filled simultaneously or in delayed chronological sequence, symmetrically or asymmetrically to one another, and thus compress the hollow organ circularly.

3. The muscle closing prosthesis system according to Claim 1,
**characterized by** elastic, compressible filler cushions, e.g., foam, which may be introduced to reduce the compression volume inside the compression cuff (1) molded as a hollow body

4. The muscle closing prosthesis system according to Claims 1 through 3,
**characterized by** the reservoir cuff (2) attached to the exterior of the support ring which comprises one or more molded hollow chambers connected to one another.

5. The muscle closing prosthesis system according to Claims 1 through 4,
**characterized by** the reservoir cuff (2) molded as a hollow body which may be protected on all sides by a flexible envelope connected to the support ring, which is not directly connected to this hollow body, however, and thus may not be evacuated in the scope of the deflation of the hollow body and is thus used as a tissue expander.

6. The muscle closing prosthesis system according to Claims 1 through 5
**characterized by** at least one or more valves are co-located between the compression cuff (1) and the reservoir cuff(2) to stop the fluid transmission or are integrated because of the design of the micropump (4).

7. The muscle closing prosthesis system according to Claim 1,
**characterized by** the micropump (4) which does not have to be integrated directly in the prosthesis, but rather may be transferred into another anatomical space in arbitrary construction, bidirectionally or unidirectionally controlled or as a manual unidirectional pump, and in this case is connected via a transmission line (6) to the compression cuff (1) and reservoir cuff (2).

8. The muscle closing prosthesis system according to Claims 1 and 7,
**characterized in that** with a unidirectional construction of the pump, the compression liquid and/or the gas or gas mixture is pumped actively from the reservoir cuff (2) into the compression cuff (1) and the compression cuff (1) is emptied passively because of the existing pressure gradient between it and the evacuated reservoir cuff (2) and because of the pressure which is exerted by the stool column on the compression cuff (1) during defecation.

## Revendications

1. Système de prothèse de sphincter, pour ouvrir et pour fermer le canal anal ou un segment d'un organe creux,
**caractérisé en ce que**,
un ballonnet de compression (1) élastique, primairement évacué et conformé en tant que corps creux est relié sur la face intérieure d'un anneau support (3) élastique, via une micro-pompe (4) intégrée ou non intégrée de version quelconque, avec un ballonnet réservoir (2) élastique et également conformé en tant que corps creux, également primairement évacué, monté sur la face extérieure de l'anneau support, **en ce que** le ballonnet de compression (1) et le ballonnet réservoir (2), chacun conformé en tant que corps creux subissent par la voie de l'inflation et de la déflation un refoulement de matière ou une déformation, l'épaisseur de paroi restant largement constante, le fluide de transmission ou un gaz ou mélange gazeux étant susceptible d'être injecté dans le ballonnet réservoir (2) évacué via un port et étant transféré via la micro-pompe (4) entre le ballonnet réservoir (2) et le ballonnet de compression (1) et la micro-pompe (4) étant susceptible d'être activée par voie magnétique, télémétrique ou par bobine d'induction par un instrument de programmation externe, via un récepteur intercalé et à implanter séparément, qui est relié avec la prothèse via un câble ou directement par radio.

2. Système de prothèse de sphincter selon la revendication 1, **caractérisé en ce que**,
le ballonnet de compression (1) se présentant en tant que corps creux moulé peut être conçu sur la face intérieure de l'anneau support, sous forme convexe ou concave avec la surface dirigée vers l'organe creux à comprimer et consiste dans deux ou dans plusieurs compartiments individuels en liaison mutuelle, qui peuvent se remplir simultanément ou selon une séquence décalée dans le temps, de façon symétrique ou asymétrique l'un contre l'autre.

3. Système de prothèse de sphincter selon la revendication 1,
**caractérisé en ce que**, pour la réduction du volume de compression, on peut introduire à l'intérieur des ballonnets de compression (1) conformés en tant que corps creux des coussins de remplissage élastiques, compressibles, par exemple de la mousse synthétique.

4. Système de prothèse de sphincter selon la revendication 1 à 3,
**caractérisé en ce que** le ballonnet réservoir (2) monté sur la face extérieure de l'anneau support consiste dans un ou dans plusieurs compartiments creux moulés, en liaison mutuelle.

5. Système de prothèse de sphincter selon la revendication 1 à 4,
**caractérisé en ce que** le ballonnet réservoir (2) formé en tant que corps creux peut être protégé de toute part par une enveloppe flexible et reliée à l'anneau support, qui n'est toutefois pas en liaison directe avec ledit corps creux et qui de ce fait ne peut pas être évacuée dans le cadre de la déflation et qui sert de ce fait d'expanseur de tissu.

6. Système de prothèse de sphincter selon la revendication 1 à 5,
**caractérisé en ce que**, entre le ballonnet de compression (1) et le ballonnet réservoir (2) sont disposées au moins une ou plusieurs valves pour fermer la transmission de fluide, ou du fait de la version de la micro-pompe (4), elles sont intégrées dans celle-ci.

7. Système de prothèse de sphincter selon la revendication 1,
**caractérisé en ce que** la micro-pompe (4) ne doit pas être obligatoirement intégrée dans la prothèse, mais **en ce qu'**elle peut être commandée en version quelconque bidirectionnelle ou en unidirectionnelle ou être délocalisée en tant que pompe manuelle unidirectionnelle dans une autre cavité anatomique et est reliée dans ce cas via un conduit de transmission (6) avec le ballonnet de compression (1) et le ballonnet réservoir (2).

8. Système de prothèse de sphincter selon la revendication 1 à 7,
**caractérisé en ce que**, dans le cas d'une version unidirectionnelle de la pompe, le liquide de compression est activement pompé du ballonnet réservoir (2) dans le ballonnet de compression (1) et **en ce que** le vidage du ballonnet de compression (1) s'effectue passivement, du fait du gradient de pression existant entre ce dernier et le ballonnet réservoir (2) évacué, ainsi que du fait de la pression qui pendant la défécation est exercée par les selles sur le ballonnet de compression (1).
